# EUROPEAN PATENT SPECIFICATION

(11) **EP 3 061 485 B1**
(45) Date of publication and mention of the grant of the patent: **25.07.2018**
(21) Application number: 15002946.0
(22) Date of filing: 15.10.2015
(51) Int. Cl.: A61M 25/00

(54) **INTRAVASCULAR CATHETER FOR INVASIVE DIAGNOSTICS OF PERIPHERAL VESSELS**
INTRAVASKULÄRER KATHETER ZUR INVASIVEN DIAGNOSE PERIPHERER GEFÄSSE
CATHÉTER INTRAVASCULAIRE POUR DIAGNOSTICS INVASIFS DE VAISSEAUX PÉRIPHÉRIQUES

(30) Priority: 25.02.2015 PL 41138015
(43) Date of publication of application: 31.08.2016
(73) Proprietor: Innovations for Heart and Vessels Sp. z o.o., 40-171 Katowice (PL)
(72) Inventor: Milewski, Krzysztof, 40-748 Katowice (PL); Buszman, Pawel, 40-748 Katowice (PL); Buszman, Piotr, 40-761 Katowice (PL)
(74) Representative: Lukaszyk, Szymon

(56) References cited:
- EP-A1- 0 766 977
- WO-A1-95/05862
- US-A- 5 843 050
- US-A1- 2012 136 320
- US-A1- 2014 128 774

## Description

The object of the invention is an intravascular catheter for invasive diagnostics of peripheral vessels. The invention relates to the field of medical devices used for diagnosis of the circulatory system, the diagnosis consisting in intravascular, i.e. intraarterial or intravenous, administration of a contrast agent under the control of X-ray (angiography, venography). This is currently one of the basic methods allowing for visualisation of narrowing changes and anatomical abnormalities of blood vessels, e.g. coronary vessels.

This method, particularly in the case of (coronary) angiography, requires the introduction of a special catheter into an artery, through an arterial sheath incorporated into one of the peripheral arteries, usually femoral, radial or brachial artery. The catheter is introduced through a sheath, usually using a guide wire or, less frequently, directly. The first of the said methods increases the safety of the procedure by minimising the risk of damage to the vessel wall by the catheter tip. Another method of increasing the safety of the procedure and at the same time of increasing its effectiveness is the use of a catheter having carefully selected materials, appropriately adjusted shape and optimal location of holes, through which the contrast is administered under high pressure. Known peripheral catheters, which are introduced using a guide wire comprise a hole at the top of the catheter, through which the contrast comes out. This situation poses a particular risk in cases where a hole of the catheter is directly adjacent to the arterial wall - then the contrast administered under high pressure can cause damage to the artery (its dissection). Another risk could also be the administration of the contrast under high pressure in the place of a peripheral atherosclerotic plaque, which can cause detachment and distal embolisation thereof. In addition, the contrast administered in this manner does not provide optimal visualisation of the target vessel, and thus the operator is usually forced to re-administer the contrast.

From European patent applications EP0453008A1 and EP0609950A1, catheters for angiography are known which are provided with several opposite holes on the periphery of the catheter and with a hole at the top of the catheter.

From US application US5085635A, a catheter provided with holes on the side walls of the catheter is known, wherein at the top of the catheter, there is a closable opening in the form of a tricuspid valve thanks to which the catherer is leakproof when introducing the guide wire through this valve. The tip of the catheter is not atraumatic.

Publication WO 95/05862 discloses a catheter comprising a hollow lumen having a distal end provided with a tip for insertion into a blood vessel, a plurality of peripherally spaced holes closely adjacent the tip, the tip having a terminal aperture capable of adopting two configurations, namely an enlarged wire carrying configuration in which it is capable of receiving and passing a wire, and a restricted configuration in which it is of substantially smaller cross-sectional area than in the enlarged condition, the aperture being normally biased into said restricted configuration. The aperture may comprise a slit or a plurality of slits or a peripheral restrictor in the form of an annulus or a plurality of restrictor members of resilient material such as latex or silicone rubber.

The aim of the present invention is to construct an improved catheter with an atraumatic tip, as described in claim 1, which will ensure the safety during the entire procedure of angiography, particularly when it passes through tortuous, calcified and atherosclerosis-affected vessels and when administering the contrast, as well as will enable its optimal outflow, and thus visualisation of the vessel in cases where the tip of the catheter abuts an artery wall. The aim of the invention is also to develop the construction of the atraumatic tip in such a way that it would be possible to use a guide wire and to handle it freely.

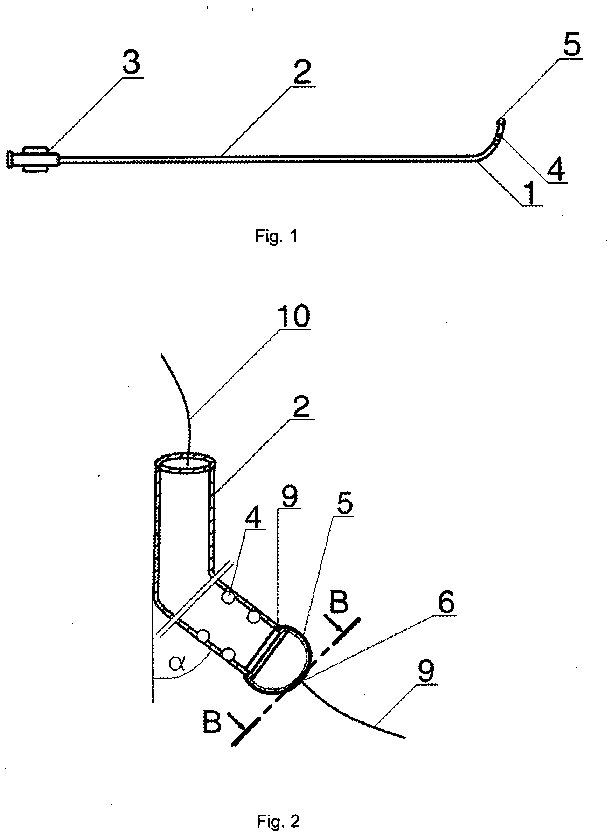

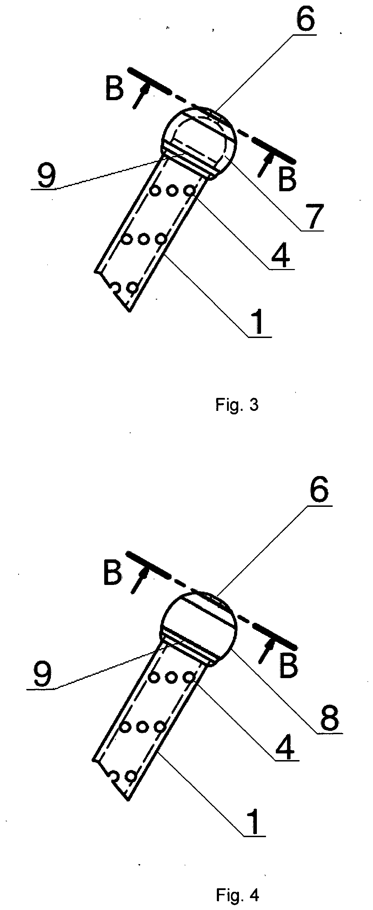

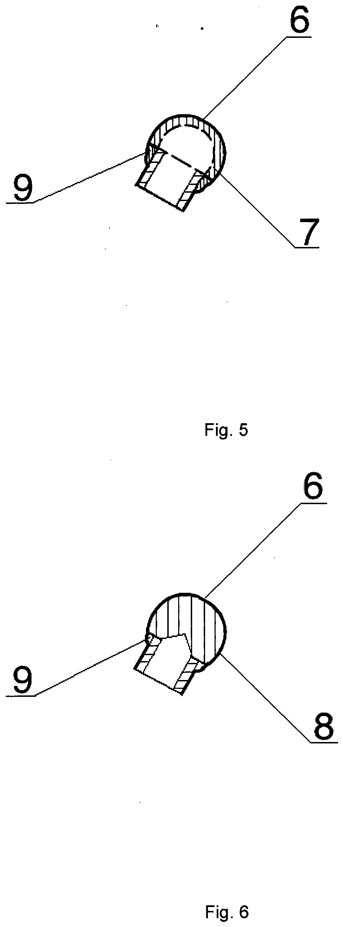

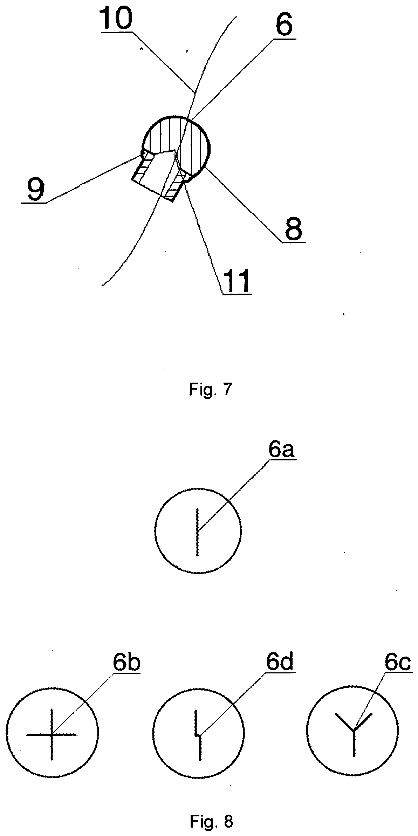

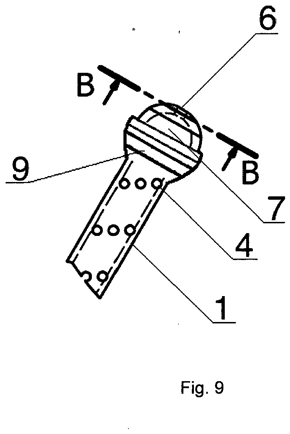

The object of the invention in an exemplary embodiment is illustrated in the drawing, in which Fig. 1 shows an overall view of the catheter with its main parts, Fig. 2 shows a catheter shaft and a distal part of the catheter, bent with respect to the shaft axis at an angle of α, Fig. 3 shows the distal part of the catheter with an atraumatic tip in a first embodiment in a spherical form, and Fig. 4 shows the distal part of the catheter with an atraumatic tip in a second embodiment in a ball-shaped form, and Fig. 5 shows a cross-section of the distal part of the catheter from Fig. 3, and Fig. 6 shows a cross-section of the distal part of the catheter from Fig. 4, and Fig. 7 shows the cross-section from Fig. 6 with a visualised guide wire passing through the valve of the atraumatic tip, Fig. 8 shows the shape of the passage (overflow) valve at the top of the atraumatic tip of the catheter, in various embodiments, as cross-section B-B of the tip, depicted in Figs. 2, 3 and 4, whereas Fig. 9 shows the atraumatic tip of the catheter in a variation where it is mounted inside the catheter tube.

An intravascular catheter for invasive diagnostics of peripheral blood vessels in an exemplary embodiment consists of successive parts: a distal part **1,** a shaft **2** and a proximal part **3,** wherein the distal part **1** is bent (folded) relative to the shaft **2** at an angle α higher than 0° and lower than 90°, ranging from 1 to 89°, preferably in the range of 10 to 80° or more preferably ranging from 30 to 60°. Along the catheter, there is a common lumen of the catheter, through which fluids are delivered and/or a guide wire **10** is introduced. The distal part of the catheter is provided with at least two holes **4** located in the side wall of the catheter, wherein these holes communicate with the lumen of the catheter to ensure outflow of the administered contrast agent outside the catheter. Preferably, the distal part comprises 2 to 20 holes, e.g. 4, 6, 8, 10 or more holes, wherein, at least two of them are located oppositely. The catheter is provided with an atraumatic rounded (radiused) tip **5** comprising, in its front space, a flexible passage (overflow) valve **6** in the form of a slot connecting, if necessary, the inner lumen of the catheter with the outside space beyond the catheter. The overflow valve **6** has the form of a through intersection at the top (most distal) wall of the atraumatic tip **5** which may have the shape of a spherical head **7,** i.e. have a rounded form unfilled inside, or have the shape of a ball-shaped head **8** filled partially or fully with the material of which it is made. The valve **6** closes the internal space of the catheter lumen through which a guide wire **10** can be introduced. The guide wire **10,** with the use of an appropriate force, can be moved through the catheter lumen and further through the valve **6** to the space outside the catheter, or retracted from the vessel lumen through the valve **6.** If the atraumatic tip comprises the ball-shaped head **8,** it is, from the side of the catheter lumen, concave, i.e. profiled e.g. in the form of a conical or funnel-shaped cavity. The thus-formed guiding recess **11** directs the guide wire into the central part of the head **8,** where the valve is located. The atraumatic tip **5** is integrated with the walls of the catheter through a joint **9** which may be an adhesive-bonded joint or welded joint, or other known one from the prior art used in this type of solutions. This joint can be formed of the same material as the atraumatic tip **5.** The valve **6** may, for example, in its cross-section, have the form of a straight line **6a** or similar to the shape of letter X - **6b,** letter Y - **6c,** or Z - **6d.**

The catheters for angiography are generally known medical devices, and for the skilled person, it will be obvious how the proximal part of the device and the shaft thereof should be constructed. The proximal part of the catheter may be a typical tip with one entrance or be branched, with two entrances, in an arrangement resembling letter "Y". With such a construction, the central (axial) entrance in the proximal tip will be intended for the guide wire, and the side entrance will be used for administering the contrast. In this exemplary embodiment, the entrance for the guide wire must be provided with a proximal valve (valve of the guide wire) which will not allow the outflow of the contrast when the guide wire was left in the catheter and, at the same time, the contrast started to be administered. Modifications of this type in the proximal part of the catheter are foreseeable by the skilled person, and the present invention covers this type of variants of the invention.

Furthermore, it is known that the internal lumen of the catheter must be communicated with holes arranged on the periphery of the catheter so that it is possible to deliver a contrast agent. The essence of the catheter according to the invention is the construction of the distal part of the catheter, i.e. curvature of the distal part of the catheter relative to the shaft, arranging, in the catheter, holes for delivering a contrast agent only on the periphery of the catheter, on side walls of the distal part, and providing the catheter with an atraumatic tip with an passage (overflow) valve.

In a first exemplary embodiment, the catheter may comprise a distal part and a catheter shaft which are aligned relative to each other.

In a second variant, the distal part is slightly bent relative to the catheter shaft, which allows for a slightly arcuate profiling of the distal part, which is shown in Fig. 1. Such a construction of the catheter is preferable because it is easier to handle it when being introduced into curved blood vessels or their bifurcations.

Terms referring to the the setting of the distal part of the catheter at an angle relative to the catheter shaft must be understood generally, as any bending or curvature of a catheter part so that its distal part is not oriented in parallel to the catheter shaft (in one line). Value of the angle indicated in Fig. 2 (the angle of bending of the distal part relative to the longitudinal axis of the shaft) refers to the relative arrangement of the two parts of the catheter in the section where there is an intentionally designed deflection of the distal part from the catheter shaft. In a further section of the catheter, approaching towards the atraumatic tip, a further, partially uncontrolled curvature of the catheter or even its arrangement in the form of a loop is possible.

The holes, with which the catheter is provided in the distal part, have a diameter of 0.010" (0.25 mm) to 0.050" (1.27 mm) and at least part of the holes is disposed oppositely to mutually overcome the pressure of streams of the fluid coming out of individual holes. The holes may be arranged in different ways, e.g. linearly along the catheter or diagonally (or helically) as shown, for example, in Figs. 2 and 3. At least two holes are arranged oppositely on the periphery of the catheter. Preferably, the holes are arranged oppositely in pairs. The holes are formed in the wall of the catheter in a direction substantially perpendicular with respect to the catheter lumen.

The atraumatic tip of the catheter is rounded and covered with a soft and safe material used in such cases, e.g. a hydrophilic material, facilitating passage of the catheter through tortuous sections of vessels. At the tip of the catheter, i.e. at the top of the atraumatic tip, preferably in a central position, in the axis of the catheter lumen, there is a valve allowing the entry and exit of a diagnostic guide wire having a diameter of 0.032" (0.81 mm) to 0.038" (0.96 mm).

The valve, due to its specific construction, remains closed, and blocks the outflow of the contrast, also under conditions of the guide wire passing through this valve. The valve is in the form of a flexible slot, i.e. a cut formed in the elastic material of the atraumatic tip. The slot is formed throughout the whole thickness of the atraumatic tip so that communication between the internal lumen of the catheter and the outer space is possible through this valve. The material forming the valve (surrounding the pass-through slot) is made of a flexible plastic, e.g. of polyurethane, polyvinyl chloride or teflon, which guarantees automatic closure of the valve after removal of the guide wire through it, but also, if necessary, withdrawal (partial opening) under the pressure of the pushed guide wire. At the time of introducing or removing the guide wire, flexible edges of the slot tightly surround the guide wire and automatically seal the space of the valve. The valve prevents the outflow of the fluid from the catheter when it is at rest and also when the guide wire is passed through the valve.

The above functionality of the valve can be realised, for example, by the use of a guide wire having a properly selected rigidity so that it is possible to squeeze it through the valve from the interior of the catheter. The guide wire having too soft tip will not be able to squeeze through the valve. However, the use of guide wires with a soft tip is possible in another embodiment of the invention, when a proximal tip with two entrances is used. Then, the other, side entrance will be dedicated to the administration of contrast, and the central entrance will serve to operate the guide wire left in the catheter from the beginning of the procedure. This entrance must be protected by a valve of the guide wire, i.e. a flexible valve (proximal valve of the catheter). The valve may for example be made of a similar material and have a structure similar to that of the valve of the atraumatic tip, except that it is not necessary to profile this valve to an atraumatic form, it can be in the form of a flat membrane. This embodiment allows the guide wire to be left in the catheter when performing administration of the contrast. It is preferable that at that time the position of the catheter can be easily corrected when, e.g., the catheter tip is moved under the pressure of the contrast administered.

Term spherical or ball-shaped head - for purposes of the present specification - should be understood as a simplified name for a shape similar to a ball or a sphere or comprising a part of a ball or a sphere. This shape can also be somewhat elongated, pear-shaped or cylindrical, comprising a spherical or ball-shaped part. The spherical head comprises an empty space in its interior so that the wall of the head is everywhere of the same or similar thickness. In such an embodiment of the invention (shown in Figs. 2, 3 and 5), the internal space of the head constitutes an extension of the internal lumen of the catheter and has a similar diameter. In another embodiment of the invention (shown in Figs. 4 and 6), the atraumatic tip has the form of a ball-shaped head, i.e. filled inside so that the interial lumen of the catheter ends at the place where the ball-shaped head is mounted. The wall of the head, as compared to the spherical shape, in the top (front) part is thicker and thus the valve in the form of a slot is more effective because it is formed by cutting a thicker layer of flexible material of the head. Preferably, the ball-shaped head comprises, from the side of the catheter lumen, a hollow, concave profiling shown in Fig. 6. It allows precise guidance of the guide wire to the place where there is a valve of the atraumatic tip, which is shown in Fig. 7.

The atraumatic tip of the catheter may be formed by mounting the head in the catheter tube and by connecting these two elements with an adhesive-bonded and/or welded joint. In this variant, shown for example in Fig. 3 and 4, the edges of the catheter tube are in contact with the head mounted thereon. In another embodiment of the invention, the spherical or ball-shaped head may be placed partially inside the catheter tube, as shown in Fig. 9. The atraumatic tip may be formed in such a manner that the joint used may cover 10 to 90% of surface of the head contained in the atraumatic tip.

The atraumatic head may have an outer diameter equal to the outer diameter of the catheter shaft, and also this head may have a diameter larger by about 10%, e.g. in the range of 5 to 20% with respect to the outer diameter of catheter shaft. The increased diameter of the catheter head, in the course of introducing the catheter, prevents accidentally placing it in very narrow blood vessels which could be damaged under the pressure of the outflowing contrast fluid.

Statement that the overflow valve has the shape of a straight line or a broken line, preferably the shape of letter T, V, X, Y or Z, should be understood rather broadly, as an approximate determination of the spatial form of the slot formed in the catheter tip. The form of a broken line may be more preferable because of the additional difficulty to open out such a slot while passing the guide wire therethrough. This ensures greater tightness of the valve.

The described construction of the catheter prevents damage to blood vessels because it contains the atraumatic tip. At the same time the tip is provided with a valve which does not interfere with the atraumatic, smooth end of the catheter, i.e. does not require any special modifications on the top surface of the catheter. The valve allows the guide wire to be passed, but during such activities of the operator, the valve remains closed. The holes in the catheter are formed in side walls, which enable a uniform introduction of a contrast fluid into the vessels. In contrast to known solutions, parallel outflow of the fluid from the top of the catheter is eliminated. Closed top (head) of the catheter allows for a uniform lateral outflow of the contrast, without disturbances which might be caused by the outflow in the axial direction. The construction of the catheter is universal and allows its use from virtually every known vascular access. The valve prevents leakage but also improves the amount of contrast through the side holes, uniform pressure - the contrast goes through side holes.

### References in the drawings:

1. distal part of the catheter
2. catheter shaft
3. proximal part of the catheter
4. hole
5. atraumatic tip
6. passage valve
6a slot in a linear shape
6b slot in the shape of letter X
6c slot in the shape of letter Y
6d slot in the shape of letter Z
7. spherical head
8. ball-shaped head
9. joint of the atraumatic tip
10.guide wire
11. guiding recess
α - bending angle of the distal part of the catheter relative to the axis of the catheter shaft

## Claims

1. An intravascular catheter for invasive diagnostics of peripheral vessels, comprising a proximal part (3), a shaft (2), and a distal part (1) with a hole or holes for delivering fluid from a catheter lumen, wherein said distal part (1), optionally positioned at an angle relative to the catheter shaft (2), is provided with at least two holes (4) located in a side wall of the catheter and with an atraumatic tip (5) comprising, in its most distal part, a flexible passage valve (6) in the form of a slot, wherein said valve (6) prevents the outflow of the fluid from the catheter when it is at rest state, **characterised in that** said atraumatic tip (5) is in the form of at least partially ball-shaped head (8) having an outer diameter larger than the outer diameter of the catheter shaft (2), preferably by about 10%, more preferably in the range of 5 to 20% with respect to the outer diameter of said catheter shaft (2), wherein said at least two holes (4) are arranged oppositely on the periphery of the catheter.

2. The catheter according to claim 1, **characterised in that** said distal part (1) is positioned at an angle α in the range of 1° to 89° relative to the catheter shaft (2).

3. The catheter according to claim 2, **characterised in that** said distal part (1) is positioned at an angle α in the range of 10° to 80" relative to the catheter shaft (2).

4. The catheter according to claim 3, **characterised in that** said distal part (1) is positioned at an angle α in the range of 30° to 60° relative to the catheter shaft (2).

5. The catheter according to any one of claims 1 to 4, **characterised in that** said valve (6) is in the shape of a straight line or the shape of a broken line, preferably in the form of T, V, X, Y or Z letter.

6. The catheter according to any one of claims 1 to 5, **characterised in that** it comprises 2 to 20 holes (4) having sizes of 0.25 mm to 1.27 mm.

## Patentansprüche

1. Intravasaler Katheter für invasive Diagnostik peripherer Gefäße bestehend aus einem proximalen Teil (3), einem Schaft (2) und einem distalen Teil (1) mit einem Loch bzw. Löchern zur Verabreichung einer Flüssigkeit aus dem Katheterlumen, in dem der besagte distale Teil (1), optional in einem Winkel gegenüber dem Katheterschaft (2) angeordnet, mindestens zwei Löcher (4) an der Seitenwand des Katheters sowie eine atraumatische Spitze (5) aufweist, die ein Durchgangsventil (6) als Schlitz in ihrem am meisten distalen Teil enthält, wobei
dieses Ventil (6) das Austreten von Flüssigkeit aus dem Katheter verhindert, wenn sich dieser im Ruhezustand befindet,
**gekennzeichnet dadurch, dass**
die besagte atraumatische Spitze (5) die Form eines zumindest teilweise kugelförmigen Kopfes (8) mit einem Außendurchmesser aufweist, der größer als Außendurchmesser des Katheterschaftes (2) vorteilhaft um ca. 10% bzw. um einen Wert im Bereich von 5 bis 20% gegenüber dem Außendurchmesser des Katheterschaftes (2) ist, wobei die besagten mindestens zwei Löcher (4) gegenüberliegend am Umfang des Katheters angeordnet sind.

2. Katheter nach Anspruch 1 **dadurch gekennzeichnet, dass** der besagte distale Teil (1) gegenüber dem Katheterschaft (2) im α-Winkel im Bereich von 1° bis 89° angeordnet ist.

3. Katheter nach Anspruch 2 **dadurch gekennzeichnet, dass** der besagte distale Teil (1) gegenüber dem Katheterschaft (2) in einem Winkel im Bereich von 10° bis 80° angeordnet ist.

4. Katheter nach Anspruch 3 **dadurch gekennzeichnet, dass** der besagte distale Teil (1) gegenüber dem Katheterschaft (2) in einem Winkel im Bereich von 30° bis 60° angeordnet ist.

5. Katheter nach einem der Ansprüche 1-4 **dadurch gekennzeichnet, dass** das besagte Ventil (6) die Form einer geraden oder gebrochenen Linie, vorteilhaft T-, V-, X-, Z- bzw. Z-förmig, aufweist.

6. Katheter nach einem der Ansprüche 1-5 **dadurch gekennzeichnet, dass** dieser von 2 bis 20 Löcher (4) mit Abmessungen 0,25 mm-1,27 mm enthält.

## Revendications

1. Un cathéter intravasculaire pour diagnostics invasifs de vaisseaux périphériques, comprenant une partie proximale (3), une tige (2) et une partie distale (1) avec un ou plusieurs trous pour délivrer un fluide depuis une lumière de cathéter, dans lequel ladite partie distale (1), éventuellement positionné sous un angle par rapport à la tige de cathéter (2), est pourvu d'au moins deux trous (4) situés dans une paroi latérale du cathéter et d'une pointe atraumatique (5) comprenant, dans sa partie la plus distale partie, une vanne de passage flexible (6) en
forme d'une fente, dans laquelle
ladite soupape (6) empêche la sortie du fluide du cathéter lorsqu'il est au repos, charactérisé en ce que
ladite pointe atraumatique (5) est sous la forme d'au moins partiellement une tête en forme de boule (8) ayant un diamètre extérieur supérieur au diamètre externe de la tige de cathéter (2), de préférence d'environ 10%, plus préférablement 5 à 20% par rapport au diamètre extérieur dudit arbre de cathéter (2), dans lequel lesdits au moins deux trous (4) sont disposés de manière opposée sur la périphérie du cathéter.

2. Le cathéter selon la revendication 1, **caractérisé en ce que** ladite partie distale (1) est positionnée selon un angle α compris entre 1° et 89° par rapport à la tige de cathéter (2).

3. Le cathéter selon la revendication 2, **caractérisé en ce que** ladite partie distale (1) est positionnée selon un angle a compris entre 10° et 80° par rapport à la tige de cathéter (2).

4. Le cathéter selon la revendication 3, **caractérisé en ce que** ladite partie distale (1) est positionnée selon un angle a compris entre 30° et 60° par rapport à la tige de cathéter (2).

5. Le cathéter selon l'une quelconque des revendications 1 à 4, **caractérisé en ce que** ladite valve (6) a la forme d'une ligne droite ou la forme d'une ligne brisée, de préférence sous la forme de T, V, X, Y ou lettre Z.

6. Le cathéter selon l'une quelconque des revendications 1 à 5, **caractérisé en ce qu'**il comprend 2 à 20 trous (4) ayant des tailles de 0.25 mm à 1.27 mm.
